Europäisches Patentamt

European Patent Office

Office européen des brevets

(11) Veröffentlichungsnummer: **0 335 295**

**A2**

(12) **EUROPÄISCHE PATENTANMELDUNG**

(21) Anmeldenummer: 89105357.1

(22) Anmeldetag: 25.03.89

(51) Int. Cl.⁴: **C07C 67/29 , C07C 69/24 , C07C 69/52 , C11D 1/74 , C07C 67/26**

(30) Priorität: 30.03.88 DE 3810793

(43) Veröffentlichungstag der Anmeldung:
04.10.89 Patentblatt 89/40

(84) Benannte Vertragsstaaten:
**BE DE ES FR GB IT NL**

(71) Anmelder: **HOECHST AKTIENGESELLSCHAFT**
**Postfach 80 03 20**
**D-6230 Frankfurt am Main 80(DE)**

(72) Erfinder: **Scholz, Hans Jürgen, Dr.**
**Orthlehnerstrasse 22**
**D-8269 Burgkirchen(DE)**
Erfinder: **Stühler, Herbert, Dr.**
**Hochfellnstrasse 12**
**D-8269 Burgkirchen(DE)**
Erfinder: **Quack, Jochen Meinhard, Dr.**
**Wilhelm-Reuter-Strasse 16**
**D-6239 Eppstein/Taunus(DE)**
Erfinder: **Schuler, Wilfried**
**Fahnenstrasse 24**
**D-6250 Limburg(DE)**
Erfinder: **Trautmann, Manfred**
**5818 Chapel Creek Ct.**
**Charlotte, N.C. 28226(US)**

(54) **Verfahren zur Herstellung von Carbonsäureestern von Alkylenglykolethern und deren Verwendung.**

(57) Es werden Carbonsäureester, insbesondere Fettsäureester, mit Ethylenoxid, Propylenoxid und/oder Butylenoxid in Gegenwart einer Alkalimetall- oder Erdalkalimetall-Verbindung aus der Gruppe der Hydroxide, Oxide und Alkoholate als Katalysator bei einer Temperatur von 100 bis 200 °C unter direktem Einbau des Alkylenoxids in den Carbonsäureester umgesetzt. Die erhaltenen Carbonsäurealkylenglykoletherester werden in hoher Ausbeute und in guter Qualität erhalten. Sie eignen sich insbesondere als aktive Komponente in Waschmitteln.

## Verfahren zur Herstellung von Carbonsäureestern von Alkylenglykolethern und deren Verwendung

Die Erfindung betrifft ein Verfahren zur Herstellung von Carbonsäureestern von Alkylenglykolethern, bei dem ein Carbonsäureester der nachstehenden Formel

$$R\text{-}\overset{\overset{\textstyle O}{\|}}{C}\text{-}OR^1$$

worin R ein Alkylrest mit 1 bis 25 C-Atomen oder ein Alkenylrest mit 2 bis 25 C-Atomen und R$^1$ ein Alkylrest mit 1 bis 10 C-Atomen ist, mit Alkylenoxid aus der Gruppe Ethylenoxid, Propylenoxid und Butylenoxid in Gegenwart eines Katalysators bei einer Temperatur von 100 bis 200 °C unter direktem Einbau des Alkylenoxids in den Carbonsäureester umgesetzt wird, und die Verwendung der so hergestellten Carbonsäureester.

Ein solches Verfahren ist in der US-Patentschrift 4,022,808 und in den beiden deutschen Offenlegungsschriften 29 51 080 und 30 08 174 beschrieben. Der verwendete Katalysator besteht aus einem Halogenid oder einer metallorganischen Verbindung von Aluminium, Eisen, Titan, Zink, Zinn oder Zirkon, gegebenenfalls in Kombination mit einer organischen Stickstoffverbindung aus der Gruppe der Amine, Amide und dergleichen, oder einer weiteren metallorganischen Verbindung als Co-Katalysator. Diese Verfahren lassen in mehreren Punkten zu wünschen übrig.

So kann das Umsetzungsprodukt mit besonders unerwünschten Verbindungen verunreinigt sein, die vom eingesetzten Katalysator und Co-Katalysator stammen. Das Umsetzungsprodukt enthält auch nach einer langen Reaktionszeit und hoher Reaktionstemperatur neben dem angestrebten Carbonsäurealkylenglykoletherester eine relativ große Menge an nicht-umgesetztem Carbonsäureester, so daß das Reaktionsprodukt als solches für die beabsichtigten Anwendungen im allgemeinen nicht geeignet ist. Die destillative Trennung der beiden Verbindungen ist zwar grundsätzlich möglich, sie ist aber insbesondere im Falle der hochsiedenden Fettsäureester, wenn überhaupt, nur noch schwer zu erreichen. Schließlich läßt auch die Qualität der erhaltenen Carbonsäurealkylenglykoletherester zu wünschen übrig.

Es ist auch schon bekannt, beispielsweise aus der US-Patentschrift 2,678,935 und der britischen Patentschrift 1 050 497, Fettsäureglyceride mit Ethylenoxid oder Propylenoxid in Gegenwart alkalischer Katalysatoren, wie Alkalimetall- oder Erdalkalimetall-Hydroxide, Alkoholate oder Salze von Fettsäuren, umzusetzen. Aus den beiden Druckschriften geht hervor, daß bei dieser Umsetzung neben dem Fettsäureglycerid auch eine mehr oder weniger große Menge von einer aktiven, Wasserstoffatome enthaltenden Verbindung anwesend ist. Daraus geht weiter hervor, daß die in Rede stehende Umsetzung nicht unter direkten oder unmittelbaren Einbau des Ethylenoxids in das Fettsäureglycerid abläuft, sondern auf Umesterungsreaktionen beruht, wie sie in der genannten US-Patentschrift 2,678,935 angegeben sind.

Die Aufgabe der Erfindung besteht darin, das eingangs genannte Verfahren in der Weise zu verbessern, daß die angegebenen Nachteile nicht auftreten, daß also insbesondere ein hoher Umsetzungsgrad zwischen Carbonsäureester und Alkylenoxid und eine hohe Ausbeute an gewünschtem Carbonsäurealkylenglykoletherester in guter Qualität erreicht wird, so daß das Umsetzungsprodukt als solches für die beabsichtigten Verwendungen gut geeignet ist.

Diese Aufgabe wird erfindungsgemäß dadurch gelöst, daß man die Umsetzung von Carbonsäureester mit Alkylenoxid unter direktem Einbau des Alkylenoxids in den Carbonsäureester mit einer Alkalimetall- oder Erdalkalimetall-Verbindung aus der Gruppe der Hydroxide, Oxide und Alkoholate als Katalysator durchführt. Solche alkalische Katalysatoren werden zwar auch bei der bekannten Umsetzung von Fettsäureglyceriden und Alkylenoxid eingesetzt. Bei dieser Umsetzung erfolgt aber, wie oben bereits erwähnt, kein direkter Einbau (Addition) des Alkylenoxids in das Fettsäureglyceridmolekül, so daß davon keine Anregung oder Motivation ausgeht, solche alkalische Katalysatoren auch für die Umsetzung eines Carbonsäureesters mit Alkylenoxid zu verwenden, und zwar für eine direkte oder unmittelbare Umsetzung des Esters mit dem Alkylenoxid. Dies gilt um so mehr, als gerade jener Stand der Technik, der sich mit der direkten Umsetzung von Carbonsäureestern und Alkylenoxid befaßt und vergleichsweise jung ist, Metallhalogenide und metallorganische Verbindungen als allein geeignete Katalysatoren für diese Umsetzung in Betracht zieht.

Die alkalischen Katalysatoren für das erfindungsgemäße Verfahren sind Hydroxide, Oxide und/oder Alkoholate von Alkalimetallen oder Erdalkalimetallen. Als Alkoholate sind jene von C$_1$-bis-C$_3$-Alkanolen bevorzugt. Als Alkalimetalle sind aus Zweckmäßigkeitsgründen Kalium und Natrium bevorzugt und als Erdalkalimetalle haben sich Barium und Strontium als besonders geeignet erwiesen. Die Menge an Katalysator kann in weiten Grenzen variieren. Im Falle von weniger als 0,05 Gew.-% Katalysator werden sehr lange Reaktionszeiten benötigt und eine Menge von mehr als 5 Gew.-% ist unwirtschaftlich. Die in Rede stehenden Alkali- und Erdalkalimetallverbindungen werden demnach erfindungsgemäß in einer Menge von 0,05 bis 5 Gew.-%, vorzugsweise 0,1 bis 3 Gew.-%, eingesetzt, Gewichtsprozente bezogen auf das Gewicht an Carbonsäureester. Die Alkalimetall- und Erdalkalimetall-Verbindungen können als solche oder in Form einer beispielsweise 20 bis 50 gew.%igen wäßrigen oder methanolischen Lösung eingesetzt werden.

Das Molverhältnis von Carbonsäureester zu Alkylenoxid richtet sich insbesondere nach dem Verwendungszweck der angestrebten Carbonsäurealkylenglykoletherester. Es hat sich herausgestellt, daß mit der erfindungsgemäßen Katalyse nicht nur die Addition von wenigen, sondern auch von vielen Alkylenoxid-Einheiten in hoher Ausbeute erreicht wird. Mit dem erfindungsgemäßen Verfahren können deshalb gleichsam maßgeschneiderte Carbonsäurealkylenglykoletherester über einen sehr weiten Alkylenglykolether-Anteil hergestellt werden. Die beiden Reaktionskomponenten, Carbonsäureester und Alkylenoxid, werden erfindungsgemäß im Molverhältnis von 1 : 3 bis 40, vorzugsweise 1 : 5 bis 25, eingesetzt. Von den angegebenen Alkylenoxiden werden erfindungsgemäß vorzugsweise Ethylenoxid oder Ethylenoxid und Propylenoxid addiert. Bei der Addition von Ethylenoxid und Propylenoxid beträgt das Molverhältnis der beiden, die blockweise oder statistisch addiert werden können, 2 bis 15 : 1, vorzugsweise 4 bis 10 : 1, wobei insgesamt, wie oben angegeben, 3 bis 40 mol, vorzugsweise 5 bis 25 mol, Ethylenoxid plus Propylenoxid pro mol Carbonsäureester eingesetzt werden.

Beim erfindungsgemäßen Verfahren werden vorzugsweise solche Carbonsäureester der angegebenen Formel eingesetzt, worin R ein Alkylrest oder Alkenylrest mit 7 bis 21 C-Atomen ist, insbesondere mit 10 bis 17 C-Atomen, wobei der Alkenylrest im allgemeinen 1 bis 3 Doppelbindungen besitzt, und $R^1$ ein Alkylrest mit 1 bis 4 C-Atomen ist. Das erfindungsgemäße Verfahren wird also vorzugsweise zur Umsetzung von Fettsäure-$C_1$-bis-$C_4$-Alkylester mit Alkylenoxid angewandt. Bevorzugte Vertreter sind demnach die $C_1$-bis-$C_4$-Alkylester, insbesondere die Methyl- oder Ethyleester, von Caprylsäure, Caprinsäure, Laurinsäure, Tridecansäure, Myristinsäure, Palmitinsäure, Margarinsäure, Stearinsäure, Arachinsäure, Behensäure, Lauroleinsäure, Myristoleinsäure, Ölsäure, Erucasäure, Linolsäure, Linolensäure, oder der technischen Fettsäuren, wie Cocosfettsäure, Sojafettsäure, Talgfettsäure, Tierkörperfettsäure und dergleichen.

Die erfindungsgemäße Umsetzung wird bei einer Temperatur von 100 bis 200 °C, vorzugsweise 130 bis 180 °C, durchgeführt. Unter 100 °C resultieren relativ lange Reaktionszeiten und über 200 °C kann es bereits zu einer Alkylenoxid-Zersetzung kommen. Das erfindungsgemäße Verfahren zur Umsetzung von Alkylenoxiden mit Carbonsäureestern kann kontinuierlich oder diskontinuierlich durchgeführt werden, wobei die Umsetzung bei erhöhtem Druck oder bei dem der Reaktionstemperatur entsprechenden Dampfdruck vor sich gehen kann. Zweckmäßigerweise wird ein Druck von weniger als 0,5 MPa, vorzugsweise von 0,1 bis 0,2 MPa, eingehalten. Bei diskontinuierlicher Arbeitsweise fällt der Druck mit fortschreitender Reaktion und bleibt konstant, wenn die Reaktion beendet ist. Nach beendeter Zugabe des Alkylenoxids wird das Reaktionsgemisch in der Regel noch einige Zeit bei Reaktionstemperatur gehalten, um praktisch vollständige Umsetzung zu erreichen. Da die Reaktion zwischen Alkylenoxid und Carbonsäureester exotherm verläuft, ist eine Apparatur erforderlich, die ein gutes Abführen der Reaktionswärme erlaubt. Die Umsetzung kann in der Weise vorgenommen werden, daß das Alkylenoxid, der Carbonsäureester und der Katalysator gemeinsam auf Reaktionstemperatur erhitzt werden oder daß Carbonsäureester und Katalysator vorgelegt und auf Reaktionstemperatur erhitzt werden, worauf das Alkylenoxid zugegeben wird. Das Alkylenoxid kann auf einmal, kontinuierlich oder portionsweise zugegeben werden. Um die Abwesenheit von Wasser oder ähnlichen Lösungsmitteln zu gewährleisten, wird der Carbonsäureester oder das Reaktionsgemisch aus Carbonsäureester und Katalysator sorgfältig entwässert beziehungsweise getrocknet. Dies kann beispielsweise durch Erhitzen bis auf etwa 100 °C unter Wasserstrahlvakuum erreicht werden. Die Wasserentfernung oder Trocknung ist klarerweise vor allem dann erforderlich, wenn der alkalische Katalysator in Form der oben erwähnten methanolischen oder wäßrigen Lösung eingesetzt wird. Nach Beendigung der Reaktion (die gesamte Reaktionszeit beträgt in der Regel 3 bis 10 Stunden) liegt ein Reaktionsprodukt vor, das praktisch vollständig aus dem angestrebten Carbonsäurealkylenglykoletherester besteht. Es versteht sich von selbst, daß der Carbonsäurealkylenglykoletherester in bezug auf die Alkylenglykol-Einheiten ein mehr oder weniger breites Homologengemisch darstellt. Eine Reinigung des Reaktionsproduktes ist in der Regel nicht erforderlich, weil der alkalische Katalysator bei den meisten Verwendungen des Reaktionsproduktes nicht stört und die Reaktionskomponenten vollständig umgesetzt sind. Sofern eine Reinigung notwendig oder gewünscht ist, wird sie beispielsweise durch Waschen, Destillation und dergleichen erreicht. Die in Rede stehenden Carbonsäurealkylenglykoletherester entsprechen der Formel RCO(Alkylenoxid)$_n$-OR$^1$, worin R und R$^1$ die angegebene Bedeutung haben und n für die Anzahl der addierten Alkylenoxideinheiten steht.

Das erfindungsgemäße Verfahren führt zu einem hohen Umsetzungsgrad sowohl bezüglich Alkylenoxid als auch bezüglich Carbonsäureester und damit zu einer hohen Ausbeute an gewünschtem Carbonsäurealkylenglykoletherester. Aufgrund des, wenn überhaupt, nur noch sehr geringen Restgehaltes an eingesetztem Carbonsäureester erfüllt bereits das Umsetzungsprodukt als solches praktisch alle Anforderungen, zumal auch der alkalische Katalysator kaum stört. Das Umsetzungsprodukt ist zudem geruchlos und hinsichtlich Farbe von sehr guter Qualität. Die mit dem erfindungsgemäßen Verfahren erhaltenen Carbonsäurealkylenglykoletherester eignen sich insbesondere als aktive Komponente in Waschmitteln.

Die Erfindung wird nun durch Beispiele noch näher erläutert:

Beispiel 1

251 g (1,12 mol) Laurinsäuremethylester und 7,5 g Bariumhydroxid, suspendiert in 50 ml Methanol, das sind 3 Gew.-% Bariumhydroxid, bezogen auf Carbonsäureester, wurden in einem 1-l-Glasautoklaven vorgelegt. Die Mischung wurde zur Entwässerung und Trocknung auf 90 °C erhitzt und bei dieser Temperatur unter Rühren 2 Stunden lang im Wasserstrahlvakuum gehalten. Die getrocknete Mischung wurde auf 150 °C erhitzt, worauf bei 150 bis 160 °C unter Rühren 245 g (5,57 mol) Ethylenoxid portionsweise im Zeitraum von 6 Stunden zudosiert wurden. Nach der Zudosierung des Ethylenoxids wurde die Mischung mit Stickstoff beaufschlagt und bei 150 bis 160 °C unter Rühren 2 Stunden lang zur Nachreaktion gehalten (Gesamtreaktionszeit also 8 Stunden). Das erhaltene Reaktionsprodukt wurde auf 80 °C abgekühlt und mittels Wasserstrahlvakuum vom nicht-umgesetzten Ethylenoxid befreit, wovon nur noch Spuren vorlagen. Es enthielt auch nur noch 4 Gew.-% nicht-umgesetzten Laurinsäuremethylester, bezogen auf das Gewicht des Reaktionsproduktes. Das Reaktionsprodukt, eine leicht gelb gefärbte Flüssigkeit, bestand also im wesentlichen aus Laurinsäureethylenglykolmethylester mit im Mittel 5 Ethylenoxid-Einheiten. Es wurde also sowohl bezüglich Ethylenoxid als auch bezüglich Carbonsäureester eine praktisch vollständige Umsetzung erreicht.

Beispiel 2

Analog Beispiel 1 wurden 220 g (0,91 mol) Myristinsäuremethylester und 2,7 g einer 40 gew.%igen wäßrigen Natriumhydroxidlösung, das sind 0,5 Gew.-% Natriumhydroxid, bezogen auf Carbonsäureester, vorgelegt, getrocknet und mit 280 g (6,36 mol) Ethylenoxid bei 110 bis 120 °C umgesetzt. Gesamtreaktionszeit 8 Stunden, wovon 2 Stunden Nachreaktionszeit sind. Das Reaktionsprodukt enthielt von den Ausgangskomponenten lediglich noch 2 Gew.-% nicht-umgesetzten Carbonsäureester, bezogen auf das Gewicht des Reaktionsproduktes. Es bestand also praktisch vollständig aus Myristinsäureethylenglykolmethylester mit im Mittel 7 Ethylenoxid-Einheiten.

Beispiel 3

Analog Beispiel 1 wurden 149 g (0,5 mol) Talgfettsäuremethylester und 5 g einer 30 gew.%igen methanolischen Natriummethylatlösung, das sind 1 Gew.-% Natriummethylat, bezogen auf Carbonsäureester, vorgelegt, getrocknet und mit 330 g (7,5 mol) Ethylenoxid bei 150 bis 160 °C umgesetzt. Gesamtreaktionszeit 11 Stunden, wovon 2 Stunden Nachreaktionszeit sind. Das Reaktionsprodukt enthielt von den Ausgangskomponenten lediglich noch 3 Gew.-% nicht-umgesetzten Carbonsäureester, bezogen auf das Gewicht des Reaktionsproduktes. Es bestand also praktisch vollständig aus Talgfettsäureethylenglykolmethylester mit im Mittel 15 Ethylenoxid-Einheiten.

Beispiel 4

Analog Beispiel 1 wurden 112 g (0,36 mol) Sojafettsäureethylester und 1 g Bariumoxid, suspendiert in 50 ml Ethanol, das sind 1 Gew.-% Bariumoxid, bezogen auf Carbonsäureester, vorgelegt, getrocknet und mit 367 g (8,34 mol) Ethylenoxid bei 150 bis 160 °C umgesetzt. Gesamtreaktionszeit 8 Stunden, wovon 2 Stunden Nachreaktionszeit sind. Das Reaktionsprodukt enthielt von den Ausgangkomponenten lediglich noch 0,1 Gew.-% nicht-umgesetzten Carbonsäureester, bezogen auf das Gewicht des Reaktionsproduktes. Es bestand also praktisch vollständig aus Sojafettsäureethylenglykolethylester mit im Mittel 23 Ethylenoxid-Einheiten.

Beispiel 5

Analog Beispiel 1 wurden 138 g (0,42 mol) Stearinsäureisopropylester und 2,8 g einer 40 gew.%igen wäßrigen Kaliumhydroxidlösung, das sind 0,8 Gew.-% Kaliumhydroxid, bezogen auf Carbonsäureester, vorgelegt, getrocknet und mit 464 g (10,55 mol) Ethylenoxid bei 150 bis 160 °C umgesetzt. Gesamtreaktionszeit 10 Stunden, wovon 2 Stunden Nachreaktionszeit sind. Das Reaktionsprodukt enthielt von den Ausgangskomponenten lediglich noch 1 Gew.-% nicht-umgesetzten Carbonsäureester, bezogen auf das Gewicht des Reaktionsproduktes. Es bestand also praktisch vollständig aus Stearinsäureethylenglykolisopropylester mit im Mittel 25 Ethylenoxid-Einheiten.

Beispiel 6

Durchführung wie im Beispiel 5, wobei 90,8 g (0,40 mol) Cocosfettsäuremethylester, 1,4 g einer 40 gew.%igen wäßrigen Natriumhydroxidlösung, das sind 0,6 Gew.-% Natriumhydroxid, bezogen auf Carbonsäureester, und 440 g (10,1 mol) Ethylenoxid eingesetzt wurden. Analog Beispiel 5 bestand das Reaktionsprodukt praktisch vollständig aus Cocosfettsäureethylenglykolmethylester mit im Mittel 25 Ethylenoxid-Einheiten.

Beispiel 7

Analog Beispiel 1 wurden 134 g (0,6 mol) Cocosfettsäuremethylester und 6,7 g einer 40 gew.%igen wäßrigen Natriumhydroxidlösung, das sind 2 Gew.-% NaOH, bezogen auf Cocosfettsäuremethylester, vorgelegt und getrocknet. Die Mischung wurde zunächst, wie in Beispiel 1, bei 150 bis 160 °C mit 315 g (7,15 mol) Ethylenoxid umgesetzt (portionsweise Zugabe den Ethylenoxids innerhalb von 8 Stunden und 2 Stunden Nachreaktionszeit) und anschließend bei 120 bis 130 °C, in einem Zeitraum von 2 Stunden und ebenfalls portionsweise, mit 69 g (1,28 mol) Propylenoxid, worauf die Mischung zur Nachreaktion 10 Stunden lang bei 120 bis 130 °C gehalten wurde. Das Reaktionsprodukt enthielt von den Ausgangskomponenten lediglich noch 1 Gew.-% nicht-umgesetzten Cocosfettsäuremethylester, bezogen auf das Gewicht des Reaktionsproduktes. Es bestand also praktisch vollständig aus Cocosfettsäureethylenglykolpropylenglykolmethylester mit im Mittel 12 Ethylenoxid-Einheiten und 2 Propylenoxid-Einheiten.

**Ansprüche**

1. Verfahren zur Herstellung von Carbonsäureestern von Alkylenglykolethern, bei dem ein Carbonsäureester der nachstehenden Formel

$$R-\overset{\overset{\textstyle O}{\|}}{C}-OR^1,$$

worin R ein Alkylrest mit 1 bis 25 C-Atomen oder ein Alkenylrest mit 2 bis 25 C-Atomen und $R^1$ ein Alkylrest mit 1 bis 10 C-Atomen ist, mit Alkylenoxid aus der Gruppe Ethylenoxid, Propylenoxid und Butylenoxid in Gegenwart eines Katalysators bei einer Temperatur von 100 bis 200 °C unter direktem Einbau des Alkylenoxids in den Carbonsäureester umgesetzt wird, dadurch gekennzeichnet, daß die Umsetzung mit einer Alkalimetall- oder Erdalkalimetall-Verbindung aus der Gruppe der Hydroxide, Oxide und Alkoholate als Katalysator durchgeführt wird.

2. Verfahren nach Anspruch 1, dadurch gekennzeichnet, daß die Alkalimetall-Verbindung und die Erdalkalimetall-Verbindung in einer Menge von 0,05 bis 5 Gew.-% eingesetzt wird, Gewichtsprozente bezogen auf das Gewicht an Carbonsäureester.

3. Verfahren nach Anspruch 1 oder 2, dadurch gekennzeichnet, daß Carbonsäureester und Alkylenoxid im Molverhältnis von 1 : 3 bis 40 eingesetzt werden.

4. Verfahren nach einem oder mehreren der Ansprüche 1 bis 3, dadurch gekennzeichnet, daß als Alkylenoxid Ethylenoxid oder Ethylenoxid und Propylenoxid eingesetzt werden.

5. Verfahren nach einem oder mehreren der Ansprüche 1 bis 4, dadurch gekennzeichnet, daß als Carbonsäureester solche der angegebenen Formel eingesetzt werden, worin R ein Alkylrest oder ein Alkenylrest mit 7 bis 21 C-Atomen und $R^1$ ein Alkylrest mit 1 bis 4 C-Atomen ist.

6. Verfahren nach einem oder mehreren der Ansprüche 1 bis 4, dadurch gekennzeichnet, daß als Carbonsäureester solche der angegebenen Formel eingesetzt werden, worin R ein Alkylrest oder ein Alkenylrest mit 10 bis 17 C-Atomen und $R^1$ ein Alkylrest mit 1 bis 4 C-Atomen ist.

7. Verfahren nach einem oder mehreren der Ansprüche 1 bis 6, dadurch gekennzeichnet, daß die Umsetzung bei einer Temperatur von 130 bis 180 °C durchgeführt wird.

8. Verfahren nach Anspruch 1, dadurch gekennzeichnet, daß ein Carbonsäureester der angegebenen Formel, worin R ein Alkylrest oder ein Alkenylrest mit 10 bis 17 C-Atomen und $R^1$ ein Alkylrest mit 1 bis 4 C-Atomen ist, mit Ethylenoxid oder Ethylenoxid und Propylenoxid in Gegenwart von 0,1 bis 3 Gew.-% Katalysator bei einer Temperatur von 130 bis 180 °C umgesetzt wird, wobei der Carbonsäureester und das Alkylenoxid im Molverhältnis 1 : 5 bis 25 eingesetzt wird.

9. Verwendung der nach den Ansprüchen 1 bis 8 erhaltenen Carbonsäureester von Alkylenglykolethern zur Bereitung von Waschmitteln.